# EUROPÄISCHE PATENTANMELDUNG

(11) **EP 4 094 751 A1**
(43) Veröffentlichungstag der Anmeldung: **30.11.2022**
(21) Anmeldenummer: 22167843.6
(22) Anmeldetag: 12.04.2022
(51) Int. Cl.: A61K 8/34, A61K 8/37, A61K 8/42, A61Q 5/12

(54) **HAAR-KOSMETISCHE ZUBEREITUNG ZUR KONDITIONIERUNG DER HAARE I**

(30) Priorität: 12.05.2021 DE 102021204835
(71) Anmelder: Beiersdorf AG, 20245 Hamburg (DE)
(72) Erfinder: Saß, Viola, 25436 Tornesch (DE); Reiter, Katharina, 21357 Barum (DE)
(74) Vertreter: Beiersdorf AG

(57) **Zusammenfassung**

Conditioner-Zubereitung für die Haare, die Silikon-frei sind, gleichwohl aber eine sehr gute Pflegeleistung aufweisen und eine angenehme Textur haben.

## Beschreibung

Die vorliegende Erfindung beschreibt Conditioner für die Haare, die Silikon-frei sind, gleichwohl aber eine sehr gute Pflegeleistung aufweisen und eine angenehme Textur haben.

Conditioner, auch als Haarspülungen bezeichnet, werden in der Regel nach der Reinigung der Haare angewendet. Conditioner sind an sich bekannt und sollen das Haar nach der Haarwäsche pflegen. Aus der großen Zahl an Dokumenten des Standes der Technik seien hier nur wenige als Beispiel genannt.

Das Dokument EP 2138156 A1 offenbart Haar-konditionierende Mittel, die verschiedene kationische Tenside in einem bestimmten Verhältnis enthalten.

Im Dokument EP 0786250 A1 werden u.a. Haarspülungen beschrieben, die ein Alkylamidoamin, Esterquats und Fettalkohole enthalten.

Das Dokument DE 102017212403 A1 offenbart Haarpflegemittel, die neben ausgewählten Alkanen Amidoamine enthalten.

Durch eine Haarwäsche wird das Haar gereinigt, d.h. es werden Schmutzpartikel und/oder -filme, die von außen auf das Haar gelangt sind, entfernt. Gleichzeitig wird aber auch die äußere Schicht des Haares, die Cuticula, angegriffen. Die Cuticula weist auf der Oberfläche kovalent gebundene Fettsäuren auf, die zur Hydrophobizität der Cuticula beitragen. Durch den Waschvorgang können zumindest zu einem gewissen Teil diese Fettsäuren ausgewaschen werden. Dieser Prozess kann bewirken, dass das Haar trocken wird.

Um diesem Phänomen entgegenzuwirken, verwenden viele Verbraucher/innen einen Conditioner nach der Haarwäsche. Der Zweck der Verwendung dieser Haar-kosmetischen Zubereitungen ist es, dem Haar idealerweise die Komponenten zurückzugeben, die möglicherweise durch die Haarwäsche mit ausgewaschen wurden. Deshalb sind im allgemeinen lipophile Komponenten in Conditionern enthalten, die mit den lipophilen Verbindungen der Cuticula wechselwirken können.

Neben der Hydrophobizität der Cuticula, ist auch die Proteinstruktur der Cuticula zu beachten. Die Zusammensetzung der Proteinstruktur resultiert in einem isoelektrischen Punkt von etwa 3,67 an der Haaroberfläche. Der pH-Wert, den die üblicherweise eingesetzten Haar-kosmetische Zubereitungen aufweisen, bewirkt, dass negativ geladene Strukturen auf der Haaroberfläche vorliegen.

Pflegende Komponenten in Conditioner-Zubereitungen umfassen also lipophile Verbindungen, die mit den lipophilen Verbindungen an der Cuticula-Oberfläche in Wechselwirkung treten und/oder positive geladene Verbindungen (kationische Verbindungen), die mit den negative geladenen Strukturen auf der Haaroberfläche wechselwirken.

In der Vergangenheit wurden vielfach Silikon-basierte Verbindungen als Pflegekomponenten in Conditioner-Zubereitungen eingesetzt. Silikone können als Silikonöle vorliegen, die hydrophob sind und mit den lipophilen Komponenten auf der Haaroberfläche wechselwirken und idealerweise einen Schutzfilm auf der Haaroberfläche ausbilden.

Silikonverbindungen können substituiert sein und zwar mit chemischen Gruppen, die positive Ladungen aufweisen (kationische Silikonverbindungen). Diese Silikonverbindungen können auch mit den negativ geladenen Strukturen an der Haaroberfläche in Wechselwirkung treten.

Neben Silikonverbindungen werden vielfach auch kationische Polymere als pflegende Komponenten eingearbeitet. Hierbei handelt es sich in der Regel um Polymere, die zumeist synthetischen Ursprungs sind. Diese kationische Pflegepolymere können ebenfalls mit den negativ geladenen Oberflächenstrukturen des Haares wechselwirken.

Die Conditioner-Zubereitungen der Vergangenheit hatten somit den Focus, die Haare zu pflegen. Der Einsatz der oben genannten großen Klassen von Pflegekomponenten kann jedoch auch Nachteile haben. Teilweise leidet die Textur dieser Zubereitungen; sie lassen sich dann nur schwer aus dem Verpackungsmittel entnehmen und schlecht im Haar verteilen. Zudem beschreiben die Verbraucher derartige Zubereitungen als "schwer", d.h. die Zubereitungen haben eine fast pastöse Textur. Ein weiterer Nachteil, der oft bei Silikon-haltigen Zubereitungen beobachtet wird, ist es, dass die Haare "beschwert" sind, d.h. der Film auf den Haaren wurde bei der Haarwäsche nicht vollständig weggewaschen und baute sich immer weiter auf. Eine Frisurgestaltung ist dann überaus schwierig.

Als weiterer Aspekt ist auch das zunehmende Umweltbewusstsein zu nennen. Der Verbraucher wünscht sich Zubereitungen, die natürliche Inhaltsstoffe enthalten und die biologische Abbaubarkeit soll auch gegeben sein. Silikonverbindungen und viele kationische Polymere sind jedoch synthetischer Natur und weisen teilweise eine schlechte biologische Abbaubarkeit auf.

Um die oben genannten Nachteile zu überwinden und zunehmend umwelt-freundlichere Conditioner-Zubereitungen zur Verfügung zu stellen, die eine leichte Textur aufweisen und frei von Silikonverbindungen, synthetischen kationischen Pflegepolymeren und alkoxylierten Verbindungen sein sollten, wurden neue Conditioner-Zubereitungen entwickelt und beschrieben, siehe dazu die PCT-Anmeldungen mit den Anmeldenummern PCT/CN2019/121114 und PCT/CN2019/121115. Beide Zubereitungen enthalten eine spezielle Mischung von Fettalkoholen, bestimmte Emollienzien und ausgewählte Emulgatoren.

Produkte gemäß der genannten Erfindungen wurden als leicht empfunden, beziehungsweise ließen sich schnell ausspülen.

Für Verbraucher, speziell mit strapazierten, geschädigten Haaren waren diese Produkte jedoch nicht befriedigend in Bezug auf pflegenden Eigenschaften.

Die Aufgabe der vorliegenden Erfindung bestand nun darin, Conditioner-Zubereitungen für die menschlichen Haare zur Verfügung zu stellen, die zum einen Silikon-frei sein, eine ansprechende Textur haben und weitgehend umweltfreundlich sein sollten und zum anderen eine hinreichende Pflegeleistung erbringen sollten. Es galt nun beide, an sich gegenläufigen, Ziele in einer Zubereitung zu verwirklichen.

Überraschenderweise konnte dies durch eine kosmetische Conditioner-Zubereitung für die menschlichen Haare, insbesondere strapazierte, geschädigte Haare, enthaltend
- wenigstens zwei Fettalkohole, wobei die wenigstens zwei Fettalkohole ≤ 16 C-Atome aufweisen und mit einem Gesamtgehalt von 2,0 bis 10,0 Gew.-%, bezogen auf das Gesamtgewicht der Zubereitung, vorliegen,
- eine Kombination von Esterölen, wobei ein oder mehrere Esteröl(e) Ester von einwertigen Alkoholen mit Fettsäuren sind und ein oder mehrere Esteröl(e) Ester von mehrwertigen Alkoholen mit Fettsäuren sind und
- wenigstens ein Alkylamidoamin,
verwirklicht werden.

Die erfindungsgemäße Zubereitung ist eine wässrige Zubereitung, vorteilhaft beträgt der Wassergehalt von 60 bis 95 Gew.-%, insbesondere von 80 bis 90 Gew.-%, bezogen das Gesamtgewicht der Zubereitung.

Die erfindungsgemäße Zubereitung liegt vorteilhaft in Form einer Emulsion vor, insbesondere in Form einer O/W-Emulsion.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-ÖI-Emulsion (W/O-Emulsion, z.B. Butter) handelt es sich um das umgekehrte Prinzip, wobei der Grundcharakter hier durch das Öl bestimmt wird.

Emulsionen benötigen zu ihrer Bildung und zur Stabilisierung im Allgemeinen einen oder mehrere Emulgatoren, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Der erfindungsgemäßen Zubereitung werden keine Emulgatoren "im klassischen Sinne" zugesetzt. Die erfindungsgemäße Zubereitung wird durch kationische Tenside stabilisiert, die in der erfindungsgemäßen Zubereitung zusätzlich die Funktion von Emulgatoren haben.

Die erfindungsmäße Conditioner-Zubereitung enthält eine Kombination von Esterölen. Esteröle können den Emollientien zugerechnet werden. Unter Emollientien werden Substanzen verstanden, die das Haart weich und geschmeidig machen. Es handelt sich dabei vorteilhaft um lipophile Komponenten.

Im allgemeinen sind Esteröle Ester von gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen ausgewählt werden. Trotz des Begriffs Esteröl können diese Substanzen flüssig oder fest sein.

Erfindungsgemäß enthält die Conditioner-Zubereitung eine Kombination von Esterölen, wobei zum einen ein oder mehrere Esteröl(e) in Form von Estern gebildet aus einem einwertigen Alkohol mit einer Fettsäure (Gruppe A) und zum zweiten ein oder mehrere Esteröl(e) in Form von Estern gebildet aus einem mehrwertigen Alkohol mit einer Fettsäure (Gruppe B) ausgewählt werden.

Die Alkohole, die zur Ausbildung der Ester der Gruppe A verwendet werden, sind einwertige Alkohole mit 2 bis 24 Kohlenstoffatomen, bevorzugt mit 2 bis 10 Kohlenstoffatomen. Besonders bevorzugt ist es, wenn die einwertigen Alkohole aus verzweigten Alkoholen mit 2 bis 10 Kohlenstoffatomen ausgewählt werden.

Die Fettsäuren, die zur Ausbildung der Ester der Gruppe A verwendet werden, sind Fettsäuren mit 6 bis 30 Kohlenstoffatomen, bevorzugt mit 6 bis 18 Kohlenstoffatomen. Besonders bevorzugt ist es, wenn unverzweigte und gesättigte Fettsäuren mit 6 bis 18 Kohlenstoffatomen gewählt werden.

Eine insbesondere bevorzugte Gruppe der Gruppe A umfasst, weiter insbesondere besteht aus, Isopropylmyristat, Isopropylpalmitat, Isopropylstearat, 2-Ethylhexylpalmitat, 2-Ethylhexyllaurat, 2-Ethylhexylstearat, und/oder Isoamyllaurat. Am meisten bevorzugt sind Isoamyllaurat, Isopropylmyristat und/oder Isopropylpalmitat.

Isoamylalkohol ist beispielsweise unter dem Handelsnamen Mackaderm bei der Firma Solvay erhältlich.

Die Alkohole, die zur Ausbildung der Ester der Gruppe B verwendet werden, sind mehrwertige Alkohole mit 2 bis 10 Kohlenstoffatomen. Bevorzugt ist es, wenn die mehrwertigen Alkohole aus verzweigten Alkoholen ausgewählt werden, weiter bevorzugt ist es, wenn der Alkohol Pentaerythrit gewählt wird.

Die Fettsäuren, die zur Ausbildung der Ester der Gruppe B verwendet werden, sind Fettsäuren mit 6 bis 30 Kohlenstoffatomen, bevorzugt mit 6 bis 24 Kohlenstoffatomen. Besonders bevorzugt ist es, wenn verzweigte und gesättigte Fettsäuren mit 6 bis 24 Kohlenstoffatomen gewählt werden.

Es ist insbesondere bevorzugt, wenn der oder die Ester der Gruppe B Pentaerythritester ist/sind, weiter insbesondere solche, gebildet aus verzweigten und gesättigten Fettsäuren mit 16 bis 20 Kohlenstoffatomen. Am meisten bevorzugt ist Pentaerythrityl Tetraisostearat.

Pentaerythrityl Tetraisostearat ist beispielsweise erhältlich unter der Handelsbezeichnung Crodamol PTIS-LQ-(MV) bei der Firma Croda GmbH.

In der erfindungsgemäßen Conditioner-Zubereitung ist/sind ein oder mehrere Esteröl(e) in Form von Estern gebildet aus einem einwertigen Alkohol mit einer Fettsäure (Gruppe A) mit einem Gesamtgehalt von 0,1 bis 2,0 Gew.-%, bevorzugt 0,5 bis 1,0 Gew.-% enthalten, bezogen auf das Gesamtgewicht der Zubereitung.

In der erfindungsgemäßen Conditioner-Zubereitung ist/sind ein oder mehrere Esteröl(e) in Form von Estern gebildet aus einem mehrwertigen Alkohol mit einer Fettsäure (Gruppe B) mit einem Gesamtgehalt von 0,1 bis 2,0 Gew.-%, bevorzugt 0,3 bis 0,6 Gew.-% enthalten, bezogen auf das Gesamtgewicht der Zubereitung.

In der erfindungsgemäßen Conditioner-Zubereitung sind wenigstens zwei Fettalkohole enthalten, wobei diese Fettalkohole ≤ 16 C-Atome aufweisen und mit einem Gesamtgehalt von 2,0 bis 10,0 Gew.-%, bezogen auf das Gesamtgewicht der Zubereitung, vorliegen.

Fettalkohole sind aliphatische, langkettige, einwertige, meist primäre Alkohole. Der Begriff Fettalkohol wird in der Regel für Fettalkohole mit einer Kettenlänge von 6 bis 22 Kohlenstoffatomen verwendet. Die Kohlenstoffkette der Fettalkohole kann eine oder mehr Doppelbindungen aufweisen und/oder unverzweigt oder verzweigt sein.

Es ist bevorzugt, wenn in der erfindungsgemäßen Conditioner-Zubereitung der oder die Fettalkohol(e) unverzweigt und/oder gesättigt ist/sind, weiter bevorzugt unverzweigt und gesättigt.

Die Fettalkohole mit ≤ 16 Kohlenstoffatomen werden bevorzugt aus der Gruppe Laurylalkohol, Myristylalkohol und/oder Cetylalkohol ausgewählt, besonders bevorzugt sind Myristylalkohol und/oder Cetylalkohol.

In der erfindungsgemäßen Conditioner-Zubereitung sind die Fettalkohole mit ≤ 16 Kohlenstoffatomen bevorzugt mit einem Gesamtgehalt von 3,5 bis 5,5 Gew.-% enthalten, bezogen auf das Gesamtgewicht der Zubereitung.

In der erfindungsgemäßen Conditioner-Zubereitung ist wenigstens ein Alkylamidoamin enthalten. Alkylamidoamine können als kationische Tenside klassifiziert werden. Alkylamidoamine weisen bei den pH-Werten, die üblicherweise in Conditioner-Zubereitungen vorliegen (pH-Werte zwischen 3,5 und 4,5) eine positive Ladung auf und zudem einen langkettigen Kohlenstoffrest.

Damit weisen diese Moleküle Strukturen auf, die eine Wechselwirkung mit der Haaroberfläche begünstigen und somit können diese Moleküle einen Beitrag zur Pflegewirkung der Conditioner-Zubereitung leisten.

Bevorzugt werden Alkylamidopropyl Dimethylamine ausgewählt, die sich durch folgende allgemeine Formel darstellen lassen: 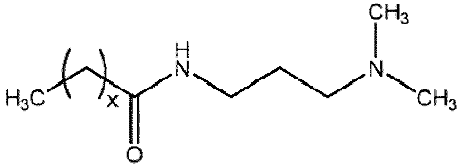 wobei x für eine ganze Zahl zwischen 14 und 22 steht. Insbesondere bevorzugt ist das Alkylamidoamin Stearamidopropyl Dimethylamin, das beispielsweise unter der Handelsbezeichnung Tego Amid S18 bei der Firma Evonok bezogen werden kann.

In der erfindungsgemäßen Conditioner-Zubereitung ist das wenigstens eine Alkylamidoamin mit einem Gesamtgehalt von 0,1 bis 5,0 Gew.-%, bevorzugt 0,3 bis 2,0 Gew.-% enthalten, bezogen auf das Gesamtgewicht der Zubereitung.

In der erfindungsgemäßen Conditioner-Zubereitung kann zusätzlich ein weiteres kationisches Tensid enthalten sein, das kein Alkylamidoamin ist. Bevorzugt wird dieses weitere kationische Tensid aus der Klasse der quartären Ammoniumverbindungen ausgewählt, die sich durch die folgende Formel darstellen lassen: 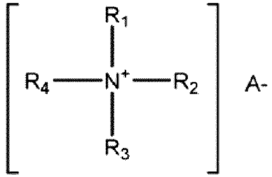 wobei die Reste R₁ R₂, R₃ und R₄ jeweils unabhängig voneinander für Wasserstoff, eine Methylgruppe oder für einen gesättigten, verzweigten oder unverzweigten Alkylrest mit einer Kettenlänge von 8 bis 30 Kohlenstoffatomen stehen, wobei mindestens einer der Reste R₁, R₂, R₃ und R₄ nicht für Wasserstoff steht, und
A- für ein physiologisch verträgliches Anion steht, beispielsweise für ein Halogenid, wie Chlorid oder Bromid sowie für Methosulfate.

Weiter bevorzugt sind drei der Reste R₁ bis R₄ Methylgruppen und ein Rest ein Alkylrest mit einer Kettenlänge von 8 bis 30 Kohlenstoffatomen. Insbesondere weiter bevorzugt wird die quartäre Ammoniumverbindung aus der Gruppe Cetrimonium Chlorid und/oder Behentrimoniun Chlorid ausgewählt.

Wenn in der erfindungsgemäßen Conditioner-Zubereitung wenigstens eine quartäre Ammoniumverbindung enthalten ist, so liegt sie mit einem Gesamtgehalt von 0,1 bis 5 Gew.-%, bevorzugt 1,0 bis 2,0 Gew.-% vor, bezogen auf das Gesamtgewicht der Zubereitung.

In der erfindungsgemäßen Conditioner-Zubereitung kann zusätzlich wenigstens ein nichtionisches Tensid enthalten sein. Es ist bevorzugt, wenn dieses wenigstens eine nichtionische Tensid aus der Gruppe der Alkylglycoside, insbesondere Alkylglucoside gewählt wird. Die genannten nichtionischen Tenside sind ungeladene Moleküle mit einem langkettigen Alkylrest. Es ist weiter bevorzugt, wenn das wenigstens eine Alkylglucosid aus der Gruppe Decylglucosid, Laurylglucosid und/oder Cocoglucosid ausgewählt wird.

Wenn in der erfindungsgemäßen Conditioner-Zubereitung wenigstens ein nichtionisches Tensid enthalten ist, so liegt es mit einem Gesamtgehalt von 0,01 bis 2,0 Gew.-%, bevorzugt 0,1 bis 0,5 Gew.-% vor, bezogen auf das Gesamtgewicht der Zubereitung.

In der erfindungsgemäßen Zubereitung kann zusätzlich wenigstens ein Moisturizer enthalten sein. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Milchsäure und/oder Lactate, insbesondere Natriumlactat, Biosaccaride Gum-1, Glycine Soja, Ethylhexyloxyglycerin, Pyrrolidoncarbonsäure und Harnstoff. Bevorzugt wird der wenigstens eine Moisturizer aus der Gruppe Glycerin, Sorbitol, Butylenglykol, Propylenglykol, Milchsäure und/oder Salze der Milchsäure ausgewählt. Es können auch zwei oder mehr verschiedene Moisturizer enthalten sind, beispielsweise Milchsäure und Glycerin.

Wenn in der erfindungsgemäßen Conditioner-Zubereitung wenigstens ein Moisturizer enthalten ist, so liegt er mit einem Gesamtgehalt von 0,1 bis 10 Gew.-%, bevorzugt 0,5 bis 5,0 Gew.-%, bezogen auf das Gesamtgewicht der Zubereitung, vor.

In der erfindungsgemäßen Zubereitung kann zusätzlich wenigstens ein Verdicker enthalten sein. Verdicker können auch als "Hydrokolloide" bezeichnet werden. Hydrokolloide sind Makromoleküle, die eine weitgehend lineare Gestalt haben und über intermolekulare und intramolekulare Wechselwirkungskräfte verfügen. Auf diese Weise kann ein netzartigen Gebilde ausgebildet. Hydrokolloide sind wasserlösliche oder Wasser-quellbare natürliche oder synthetische Polymere, die in wässrigen Systemen Gele oder viskose Lösungen bilden. Solche wasserlöslichen Polymere stellen eine große Gruppe chemisch sehr unterschiedlicher natürlicher und synthetischer Polymere dar, deren gemeinsames Merkmal ihre Löslichkeit oder Quellbarkeit in Wasser bzw. wässrigen Medien ist. Voraussetzung dafür ist, dass diese Polymere über eine für die Wasserlöslichkeit ausreichende Anzahl an hydrophilen Gruppen besitzen und nicht zu stark vernetzt sind.

Die Gruppe der kosmetisch und dermatologisch relevanten Hydrokolloide, die natürlich oder Natur-basiert sind, lässt sich wie folgt einteilen in:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- anorganische Verbindungen, wie z. B. Polykieselsäuren, Tonmineralien wie Montmorillonite, Zeolithe, Kieselsäuren.

Es ist bevorzugt, wenn in der erfindungsgemäßen Conditioner-Zubereitung der wenigstens eine Verdicker aus der Gruppe der Celluloseether, wie beispielsweise Hydroxyethyl- und -propylcellulose gewählt wird.

Wenn in der erfindungsgemäßen Conditioner-Zubereitung wenigstens ein Celluloseether enthalten ist, so liegt er mit einem Gesamtgehalt von 0,1 bis 2,0 Gew.-%, bevorzugt 0,5 bis 1,0 Gew.-% vor, bezogen auf das Gesamtgewicht der Zubereitung.

In der erfindungsgemäßen Conditioner-Zubereitung kann vorteilhaft wenigstens ein Konservierungsmittel enthalten sein. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können weiter vorteilhaft Phenoxyethanol, Methyl-, Ethyl-, Propylparaben, Benzylalkohol, Sorbinsäure und/oder Salze der Sorbinsäure, beispielsweise Kalium Sorbat, Benzoesäure und/oder Salze der Benzoesäure, beispielsweise Natrium Benzoat, Salicylsäure und/oder Salze der Salicylsäure, beispielsweise Natrium Salicylat eingesetzt werden. Es ist insbesondere vorteilhaft Phenoxyethanol einzusetzen.

Wenn in der erfindungsgemäßen Conditioner-Zubereitung wenigstens ein Konservierungsmittel enthalten ist, so liegt das wenigstens eine Konservierungsmittel mit einem Gehalt von 0,01 bis 5,0 Gew. %, bevorzugt von 0,1 bis 1,0 Gew. %, besonders bevorzugt von 0,3 bis 0,7 Gew. % in der erfindungsgemäßen Zubereitung vor, bezogen auf das Gesamtgewicht der Zubereitung.

Zusätzlich kann wenigstens eine physiologisch verträgliche anorganische Säure oder organische Säure enthalten sein. Die Säuren werden benötigt, um den pH-Wert der Zubereitung auf einen sauren pH-Wert im physiologischen Bereich einzustellen. Vorteilhaft ist ein pH-Wert-Bereich von 3,5 bis 4,5. Milchsäure wirkt als Moisturizer und gleichzeitig zur Stabilisierung des pH-Wertes.

Es ist vorteilhaft, wenn Citronensäure und/oder Milchsäure ausgewählt werden, um den pH-Wert einzustellen.

Weiterhin ist es vorteilhaft, wenn die Zubereitung ein Parfüm enthält. Parfüme sind Mischungen aus Parfümrohstoffen. Das Parfüm liegt vorteilhaft mit einem Gehalt von 0,05 bis 1 Gew.-% vor, bezogen auf das Gesamtgewicht der Zubereitung.

Die erfindungsgemäße Zubereitung kann auf jede für eine derartige Zubereitung im StdT bekannte und geeignete Weise hergestellt werden.

Der erfindungsgemäßen Conditioner-Zubereitung werden keine Silikon-Verbindungen zugesetzt, ebenso sind keine Silikon-Verbindungen enthalten, die mit an sich anderen Rohstoffen in die Zubereitung gelangen. Die erfindungsgemäße Conditioner-Zubereitung ist also frei von Silikon-Verbindungen. Silikon-Verbindungen zeichnen sich durch das Vorhandensein von Silicium- und Sauerstoffatomen aus, die miteinander verknüpft sind und eine Vielzahl verschiedener Verbindungen ausbilden können.

Weiterhin werden der erfindungsgemäßen Conditioner-Zubereitung werden keine PEG-haltigen Verbindungen zugesetzt, ebenso sind keine PEG-haltigen Verbindungen enthalten, die mit an sich anderen Rohstoffen in die Zubereitung gelangen. Die erfindungsgemäße Conditioner-Zubereitung ist also frei von PEG-haltigen-Verbindungen. PEG ist die Abkürzung für Polyethylenglycol; viele der Struktureinheiten (-CH₂-CH₂-O-) sind dabei miteinander verknüpft. Diese Strukturen können bewirken, dass eine Penetration durch die Haut bewirkt oder verstärkt wird. Unter PEG-haltige Verbindungen werden Polyethylenglykole und Polyethylenglykolderivate verstanden.

Ebenso werden den Conditioner-Zubereitung werden keine Polyquaternium-Verbindungen zugesetzt, auch sind keine Polyquaternium-Verbindungen enthalten, die mit an sich anderen Rohstoffen in die Zubereitung gelangen. Polyquaternium-Verbindungen sind polymere Verbindungen, die sich aus einem oder verschiedenen Monomeren zusammensetzen. Ihre chemische Struktur ist verschieden; das gemeinsame Merkmal ist jedoch das Vorhandensein vom Ammonium-Gruppen. Die erfindungsgemäßen Conditioner-Zubereitungen sind also frei vom Polyquaternium-Verbindungen.

Um die Pflegewirkung der erfindungsgemäßen Conditioner-Zubereitungen zu ermitteln wurde eine Zubereitung gemäß Rezepturbeispiel 1 hergestellt.

Für die Messungen wurden 7 Tressen (7-fach Bestimmung) pro Messung vorbereitet. Für die Tressen wurden jeweils 2g geschädigtes europäisches Haar verwendet. Es erfolgte ein Waschschritt mit einem Standard-Shampoo, das keine pflegenden Zusätze enthielt (Wasserhärte 1,5 bis 2,5 mmol/l CaCO₃). Für die Bestimmung der Trockenkämmkraft und der Geschmeidigkeit wurden die Tressen für 18 Stunden unter konstanten Bedingungen getrocknet (Klimaraum mit 22°C±1°C, 55% ± 5% relative Luftfeuchtigkeit). Die erste Messung der Naß- und Trockenkämmkraft als auch der Geschmeidigkeit an den Haartressen diente der Bestimmung des Basiswertes (t₀). 0,4 ml Testprodukt (erfindungsgemäßes Zubereitung) wurden auf das nasses Haar aufgetragen und wieder ausgewaschen. Für die Bestimmung der Nasskämmkraft wurde das nasse Haar vermessen. Für die Bestimmung der Trockenkämmkraft und die Bestimmung der Geschmeidigkeit wurde das Haar unter konstanten Bedingungen getrocknet (siehe oben) und anschließend vermessen. Die Ergebnisse sind in der nachfolgenden Tabelle dargestellt.

| **Zubereitung gemäß Rezepturbeispiel 1** | | | | |
|---|---|---|---|---|
| | | **t₀** | **t₁** | |
| **Nasskämmkraft [mN]** | Mittelwert | 656,2 | 117,3 | |
| | Standardabweichung | 69,9 | 9,4 | |
| | Relative Veränderung zu t₀* | | | -81,9 |
| | | | | |
| | | **t₀** | **t₁** | |
| **Geschmeidigkeit [mN]** | Mittelwert | 186,0 | 155,7 | |
| | Standardabweichung | 3,9 | 6,1 | |
| | Relative Veränderung zu t₀* | | | -16,3% |
| | | | | |
| | | **t₀** | **t₁** | |
| **Trockenkämmkraft [mN]** | Mittelwert | 9,5 | 7,1 | |
| | Standardabweichung | 0,5 | 0,6 | |
| | Relative Veränderung zu t₀* | | | -25,8% |

| | | | | |
|---|---|---|---|---|
| * [(t₁-t₀)/t₀] | | | | |

Die Ergebnisse der Messungen zeigen, dass nach Auftrag eines erfindungsgemäßen Produktes bei allen drei Messungen weniger Kraft aufgewendet werden musste, sowohl beim Kämmen als auch beim Biegen (Geschmeidigkeitsmessung) der Haare. Dies ist ein Anzeichen dafür, dass die Haaroberfläche glatter war und weniger Widerstände beim Kämmen oder Biegen auftraten. Dies ist ein Beleg dafür, dass das erfindungsgemäße Produkt eine Glättung der Haaroberfläche erzielt hat und damit ein nachweislicher Pflegeeffekt erzielt wurde.

Zusammenfassend ist die vorliegende Patentanmeldung auf den Gegenstand gerichtet, der in den folgenden, nummerierten Paragrafen beschrieben ist:
1. Kosmetische Conditioner-Zubereitung für die menschlichen Haaren, insbesondere strapazierte, geschädigte Haare, enthaltend
   - wenigstens zwei Fettalkohole, wobei die wenigstens zwei Fettalkohole ≤ 16 C-Atome aufweisen und mit einem Gesamtgehalt von 2,0 bis 10,0 Gew.-%, bezogen auf das Gesamtgewicht der Zubereitung, vorliegen,
   - eine Kombination von Esterölen, wobei ein oder mehrere Esteröl(e) Ester von einwertigen Alkoholen mit Fettsäuren sind (Gruppe A) und ein oder mehrere Esteröl(e) Ester von mehrwertigen Alkoholen mit Fettsäuren (Gruppe B) sind und
   - wenigstens eine Alkylamidoamin.
2. Zubereitung nach Paragraf 1 dadurch gekennzeichnet, dass der Wassergehalt von 60 bis 95 Gew.-%, insbesondere von 80 bis 90 Gew.-% beträgt, bezogen das Gesamtgewicht der Zubereitung.
3. Zubereitung nach Paragraf 1 und/oder 2, dadurch gekennzeichnet, dass die Zubereitung in Form einer Emulsion vor, insbesondere in Form einer O/W-Emulsion.
4. Zubereitung nach wenigstens einem der vorstehenden Paragrafen, dadurch gekennzeichnet, dass der/die Alkohol(e) in dem/den Esteröl(en) der Gruppe A einwertige Alkohole mit 2 bis 24 Kohlenstoffatomen, bevorzugt mit 2 bis 10 Kohlenstoffatomen, besonders bevorzugt verzweigte Alkoholen mit 2 bis 10 Kohlenstoffatomen sind.
5. Zubereitung nach wenigstens einem der vorstehenden Paragrafen, dadurch gekennzeichnet, dass die Fettsäure(n) in dem/den Esteröl(en) der Gruppe A Fettsäuren mit 6 bis 30 Kohlenstoffatomen, bevorzugt mit 6 bis 18 Kohlenstoffatomen, besonders bevorzugt unverzweigte und gesättigte Fettsäuren mit 6 bis 18 Kohlenstoffatomen sind.
6. Zubereitung nach wenigstens einem der vorstehenden Paragrafen, dadurch gekennzeichnet, dass das/die Esteröl(en) der Gruppe A ausgewählt werden aus der Gruppe, die Isopropylmyristat, Isopropylpalmitat, Isopropylstearat, 2-Ethylhexylpalmitat, 2-Ethylhexyllaurat, 2-Ethylhexylstearat, und/oder Isoamyllaurat umfasst, insbesondere aus Isopropylmyristat, Isopropylpalmitat, Isopropylstearat, 2-Ethylhexylpalmitat, 2-Ethylhexyllaurat, 2-Ethylhexylstearat, und/oder Isoamyllaurat besteht, am meisten bevorzugt ist/sind das/die Esteröl(en) der Gruppe A Isoamyllaurat, Isopropylmyristat und/oder Isopropylpalmitat.
7. Zubereitung nach wenigstens einem der vorstehenden Paragrafen, dadurch gekennzeichnet, dass der/die Alkohol(e) in dem/den Esteröl(en) der Gruppe B mehrwertige Alkohole mit 2 bis 10 Kohlenstoffatomen, bevorzugt mehrwertige, verzweigte Alkohole, weiter bevorzugt ist der Alkohol Pentaerythrit.
8. Zubereitung nach wenigstens einem der vorstehenden Paragrafen, dadurch gekennzeichnet, dass die Fettsäure(n) in dem/den Esteröl(en) der Gruppe B Fettsäuren mit 6 bis 30 Kohlenstoffatomen, bevorzugt mit 6 bis 24 Kohlenstoffatomen, besonders bevorzugt verzweigte und gesättigte Fettsäuren mit 6 bis 24 Kohlenstoffatomen gewählt werden.
9. Zubereitung nach wenigstens einem der vorstehenden Paragrafen, dadurch gekennzeichnet, dass das/die Esteröl(en) der Gruppe B Pentaerythritester sind, bevorzugt solche, gebildet aus verzweigten und gesättigten Fettsäuren mit 16 bis 20 Kohlenstoffatomen, besonders bevorzugt ist Pentaerythrityl Tetraisostearat.
10. Zubereitung nach wenigstens einem der vorstehenden Paragrafen, dadurch gekennzeichnet, dass das/die Esteröl(en) der Gruppe A mit einem Gesamtgehalt von 0,1 bis 2,0 Gew.-%, bevorzugt 0,5 bis 1,0 Gew.-% enthalten ist/sind, bezogen auf das Gesamtgewicht der Zubereitung.
11. Zubereitung nach wenigstens einem der vorstehenden Paragrafen, dadurch gekennzeichnet, dass das/die Esteröl(en) der Gruppe B mit einem Gesamtgehalt von 0,1 bis 2,0 Gew.-%, bevorzugt 0,3 bis 0,6 Gew.-% enthalten ist/sind, bezogen auf das Gesamtgewicht der Zubereitung.
12. Zubereitung nach wenigstens einem der vorstehenden Paragrafen, dadurch gekennzeichnet, dass die Fettalkohole aufweisend ≤ 16 Kohlenstoffatome aus der Gruppe Laurylalkohol, Myristylalkohol und/oder Cetylalkohol ausgewählt wird/werden, bevorzugt sind Myristylalkohol und/oder Cetylalkohol.
13. Zubereitung nach wenigstens einem der vorstehenden Paragrafen, dadurch gekennzeichnet, dass die Fettalkohole aufweisend ≤ 16 Kohlenstoffatome mit einem Gesamtgehalt von 3,5 bis 5,5 Gew.-% enthalten sind, bezogen auf das Gesamtgewicht der Zubereitung.
14. Zubereitung nach wenigstens einem der vorstehenden Paragrafen, dadurch gekennzeichnet, dass das wenigstens eine Alkylamidoamin mit einem Gesamtgehalt von 0,1 bis 5,0 Gew.-%, bevorzugt 0,3 bis 2,0 Gew.-% enthalten ist, bezogen auf das Gesamtgewicht der Zubereitung.
15. Zubereitung nach wenigstens einem der vorstehenden Paragrafen, dadurch gekennzeichnet, dass das Alkylamidoamin Stearamidopropyl Dimethylamin ist.
16. Zubereitung nach wenigstens einem der vorstehenden Paragrafen, dadurch gekennzeichnet, dass zusätzlich wenigstens ein weiteres kationisches Tensid enthalten ist, das kein Alkylamidoamin ist, bevorzugt wird das wenigstens eine weitere kationische Tensid aus der Gruppe der quartären Ammoniumverbindungen ausgewählt, weiter bevorzugt aus der Gruppe Cetrimonium Chlorid und/oder Behentrimoniun Chlorid.
17. Zubereitung nach wenigstens einem der vorstehenden Paragrafen, dadurch gekennzeichnet, dass die wenigstens eine quartäre Ammoniumverbindung mit einem Gesamtgehalt von 0,1 bis 5,0 Gew.-%, bevorzugt 1,0 bis 2,0 Gew.-% vorliegt, bezogen auf das Gesamtgewicht der Zubereitung.
18. Zubereitung nach wenigstens einem der vorstehenden Paragrafen, dadurch gekennzeichnet, dass zusätzlich wenigstens ein nichtionisches Tensid, bevorzugt, bevorzugt wenigstens ein Alkylglycosid, weiter bevorzugt wenigstens ein Alkylglycosid, insbesondere weiter bevorzugt ausgewählt aus der Gruppe Decylglucosid, Laurylglucosid und/oder Cocoglucosid, enthalten ist.
19. Zubereitung nach wenigstens einem der vorstehenden Paragrafen, dadurch gekennzeichnet, dass das wenigstens eine nichtionische Tensid es mit einem Gesamtgehalt von 0,01 bis 2,0 Gew.-%, bevorzugt 0,1 bis 0,5 Gew.-% vorliegt, bezogen auf das Gesamtgewicht der Zubereitung.
20. Zubereitung nach wenigstens einem der vorstehenden Paragrafen, dadurch gekennzeichnet, dass zusätzlich wenigstens ein Moisturizer, bevorzugt ausgewählt aus Glycerin, Sorbitol, Butylenglykol, Propylenglykol, Milchsäure und/oder Lactate, Biosaccaride Gum-1, Glycine Soja, Ethylhexyloxyglycerin und/oder Harnstoff, weiter bevorzugt aus der Gruppe Glycerin, Sorbitol, Butylenglykol, Propylenglykol, Milchsäure und/oder Salze der Milchsäure, enthalten ist.
21. Zubereitung nach wenigstens einem der vorstehenden Paragrafen, dadurch gekennzeichnet, dass der wenigstens eine Moisturizer mit einem Gesamtgehalt von 0,1 bis 10 Gew.-%, bevorzugt 0,5 bis 5,0 Gew. % enthalten ist, bezogen auf das Gesamtgewicht der Zubereitung.
22. Zubereitung nach wenigstens einem der vorstehenden Paragrafen, dadurch gekennzeichnet, dass zusätzlich wenigstens ein Verdicker, bevorzugt ausgewählt aus der Gruppe der Celluloseether enthalten ist.
23. Zubereitung nach wenigstens einem der vorstehenden Paragrafen, dadurch gekennzeichnet, dass der wenigstens eine Verdicker mit einem Gesamtgehalt von 0,1 bis 2,0 Gew.-%, bevorzugt 0,5 bis 1,0 Gew.-% vorliegt, bezogen auf das Gesamtgewicht der Zubereitung.
24. Zubereitung nach wenigstens einem der vorstehenden Paragrafen, dadurch gekennzeichnet, dass die Zubereitung frei von Silikon-Verbindungen ist.
25. Zubereitung nach wenigstens einem der vorstehenden Paragrafen, dadurch gekennzeichnet, dass die Zubereitung frei von PEG-haltigen-Verbindungen ist.
26. Zubereitung nach wenigstens einem der vorstehenden Paragrafen, dadurch gekennzeichnet, dass die Zubereitung frei von Polyquaternium-Verbindungen ist.

### Beispiele:

Die untenstehenden Beispiele sollen die Erfindung illustrieren und nicht einschränken. Die Angaben beziehen sich auf Gew.-% und den jeweiligen Aktivgehalt, soweit nicht anders angegeben.

| **INCI** | **Example 1** | **Example 2** | **Example 3** |
|---|---|---|---|
| Cetyl Alcohol | 3,4 | 3 | 3,8 |
| Myristyl Alcohol | 1,7 | 2 | 1,4 |
| Pentaerythrityl Tetraisostearate | 0,5 | 0,5 | 0,4 |
| Isoamyl Laurate | 0,5 | 0,5 | 0,4 |
| Stearamidopropyl Dimethylamine | 1,5 | 0,8 | 1,2 |
| Lactic Acid | 0,54 | 0,27 | 0,45 |
| Cetrimonium Chloride | 1,0 | 1,2 | |
| Behentrimonium Chloride | | | 1,3 |
| Hydroxyethylcellulose | 0,5 | 0,4 | 0,7 |
| Coco-Betaine | 0,3 | 0,2 | |
| Citric Acid | 0,25 | 0,2 | 0,5 |
| Sodium Citrate | 0,18 | | 0,35 |
| Panthenol | 0,1 | 0,3 | |
| Phenoxyethanol | 0,4 | 0,4 | 0,5 |
| Parfum | 0,7 | 0,7 | 0,6 |
| Aqua | ad 100 | ad 100 | ad 100 |

## Patentansprüche

1. Kosmetische Conditioner-Zubereitung für die menschlichen Haaren, insbesondere trockene und/oder sensible Haare, enthaltend
- wenigstens zwei Fettalkohole, wobei die wenigstens zwei Fettalkohole ≤ 16 C-Atome aufweisen und mit einem Gesamtgehalt von 2,0 bis 10,0 Gew.-%, bezogen auf das Gesamtgewicht der Zubereitung, vorliegen,
- eine Kombination von Esterölen, wobei ein oder mehrere Esteröl(e) Ester von einwertigen Alkoholen mit Fettsäuren sind (Gruppe A) und ein oder mehrere Esteröl(e) Ester von mehrwertigen Alkoholen mit Fettsäuren (Gruppe B) sind und
- wenigstens ein Alkylamidoamin.

2. Zubereitung nach Anspruch 1, **dadurch gekennzeichnet, dass** der/die Alkohol(e) in dem/den Esteröl(en) der Gruppe A einwertige Alkohole mit 2 bis 24 Kohlenstoffatomen, bevorzugt mit 2 bis 10 Kohlenstoffatomen, besonders bevorzugt verzweigte Alkoholen mit 2 bis 10 Kohlenstoffatomen sind.

3. Zubereitung nach Anspruch 1 und/oder 2, **dadurch gekennzeichnet, dass** die Fettsäure(n) in dem/den Esteröl(en) der Gruppe A Fettsäuren mit 6 bis 30 Kohlenstoffatomen, bevorzugt mit 6 bis 18 Kohlenstoffatomen, besonders bevorzugt unverzweigte und gesättigte Fettsäuren mit 6 bis 18 Kohlenstoffatomen sind.

4. Zubereitung nach wenigstens einem der vorstehenden Ansprüche, **dadurch gekennzeichnet, dass** das/die Esteröl(en) der Gruppe A ausgewählt werden aus der Gruppe, die Isopropylmyristat, Isopropylpalmitat, Isopropylstearat, 2-Ethylhexylpalmitat, 2-Ethylhexyllaurat, 2-Ethylhexylstearat, und/oder Isoamyllaurat umfasst, insbesondere aus Isopropylmyristat, Isopropylpalmitat, Isopropylstearat, 2-Ethylhexylpalmitat, 2-Ethylhexyllaurat, 2-Ethylhexylstearat, und/oder Isoamyllaurat besteht, am meisten bevorzugt ist/sind das/die Esteröl(en) der Gruppe A Isoamyllaurat, Isopropylmyristat und/oder Isopropylpalmitat.

5. Zubereitung nach wenigstens einem der vorstehenden Ansprüche, **dadurch gekennzeichnet, dass** der/die Alkohol(e) in dem/den Esteröl(en) der Gruppe B mehrwertige Alkohole mit 2 bis 10 Kohlenstoffatomen, bevorzugt mehrwertige, verzweigte Alkohole, weiter bevorzugt ist der Alkohol Pentaerythrit.

6. Zubereitung nach wenigstens einem der vorstehenden Ansprüche, **dadurch gekennzeichnet, dass** die Fettsäure(n) in dem/den Esteröl(en) der Gruppe B Fettsäuren mit 6 bis 30 Kohlenstoffatomen, bevorzugt mit 6 bis 24 Kohlenstoffatomen, besonders bevorzugt verzweigte und gesättigte Fettsäuren mit 6 bis 24 Kohlenstoffatomen gewählt werden.

7. Zubereitung nach wenigstens einem der vorstehenden Ansprüche, **dadurch gekennzeichnet, dass** das/die Esteröl(en) der Gruppe B Pentaerythritester sind, bevorzugt solche, gebildet aus verzweigten und gesättigten Fettsäuren mit 16 bis 20 Kohlenstoffatomen, besonders bevorzugt ist Pentaerythrityl Tetraisostearat.

8. Zubereitung nach wenigstens einem der vorstehenden Ansprüche, **dadurch gekennzeichnet, dass** die Fettalkohol(e) aufweisend ≤ 16 Kohlenstoffatome aus der Gruppe Laurylalkohol, Myristylalkohol und/oder Cetylalkohol ausgewählt wird/werden, bevorzugt sind Myristylalkohol und/oder Cetylalkohol.

9. Zubereitung nach wenigstens einem der vorstehenden Ansprüche, **dadurch gekennzeichnet, dass** das wenigstens eine Alkylamidoamin mit einem Gesamtgehalt von 0,1 bis 5,0 Gew.-%, bevorzugt 0,3 bis 2,0 Gew.-% enthalten ist, bezogen auf das Gesamtgewicht der Zubereitung.

10. Zubereitung nach wenigstens einem der vorstehenden Ansprüch,e **dadurch gekennzeichnet, dass** das Alkylamidoamin Stearamidopropyl Dimethylamin ist.

11. Zubereitung nach wenigstens einem der vorstehenden Ansprüche, **dadurch gekennzeichnet, dass** die Zubereitung frei von Silikon-Verbindungen ist.

12. Zubereitung nach wenigstens einem der vorstehenden Ansprüche, **dadurch gekennzeichnet, dass** die Zubereitung frei von PEG-haltigen-Verbindungen ist.

13. Zubereitung nach wenigstens einem der vorstehenden Ansprüche, **dadurch gekennzeichnet, dass** die Zubereitung frei von Polyquaternium-Verbindungen ist.
